# FASCICULE DE BREVET EUROPEEN

(11) **EP 3 328 813 B1**
(45) Date de publication et mention de la délivrance du brevet: **29.09.2021**
(21) Numéro de dépôt: 16738446.0
(22) Date de dépôt: 13.07.2016
(51) Int. Cl.: C04B 35/488, A61C 13/083, G04B 37/22, A44C 27/00, A61K 6/818, A61K 6/822

(54) **PRODUIT FRITTE A BASE D'ALUMINE ET DE ZIRCONE**
SINTERPRODUKT AUF ALUMINIUMOXIDBASIS UND ZIRKONOXIDBASIS
SINTERED ALUMINA-BASED AND ZIRCONIA-BASED PRODUCT

(30) Priorité: 30.07.2015 FR 1557315
(43) Date de publication de la demande: 06.06.2018
(73) Titulaire: SAINT-GOBAIN CENTRE DE RECHERCHES ET D'ETUDES EUROPEEN, 92400 Courbevoie (FR)
(72) Inventeur: PERIE, Thomas, 84000 Avignon (FR)
(74) Mandataire: Nony
(86) Numéro de dépôt international: PCT/EP2016/066697
(87) Numéro de publication internationale: WO 2017/016879

(56) Documents cités:
- WO-A1-2008/040813
- WO-A1-2010/140121
- WO-A1-2011/000390
- WO-A2-2011/077380
- JP-A- 2006 104 024

## Description

### Domaine technique

L'invention concerne un produit fritté à base d'alumine et de zircone, un mélange particulaire permettant d'obtenir un tel produit, ainsi qu'un procédé de fabrication dudit produit.

### Arrière-plan de l'invention

Parmi les produits réfractaires, on distingue les produits fondus et coulés et les produits frittés.

A la différence des produits frittés, les produits fondus et coulés comportent le plus souvent une phase vitreuse intergranulaire très abondante qui vient remplir un réseau de grains cristallisés. Les problèmes rencontrés dans leurs applications respectives par les produits frittés et par les produits fondus et coulés, et les solutions techniques adoptées pour les résoudre, sont donc généralement différents. Par ailleurs, du fait des différences importantes entre les procédés de fabrication, une composition mise au point pour fabriquer un produit fondu et coulé n'est pas *a priori* utilisable telle quelle pour fabriquer un produit fritté, et réciproquement.

Les produits frittés sont obtenus par mélange de matières premières appropriées puis mise en forme à cru de ce mélange et cuisson de la pièce crue résultante à une température et pendant un temps suffisants pour obtenir le frittage de cette pièce crue.

Les produits frittés, selon leur composition chimique, présentent des propriétés différentes et sont donc destinés à des industries très variées.

Parmi les produits frittés céramiques, les produits de zircone yttriée quadratique, comportant typiquement une quantité molaire d'Y₂O₃ égale à 3%, présentent une contrainte à la rupture et une dureté élevée.

Les produits de zircone cériée, comportant typiquement une quantité molaire de CeO₂ égale à 12%, présentent une ténacité très élevée, supérieure à celle des produits de zircone yttriée, mais une contrainte à la rupture et une dureté plus faibles.

Les documents WO2008/040813A1, WO2010/140121A1, WO2011/000390A1 et JP2006104024A décrivent des produits frittés de zircone stabilisée.

Le document WO2011/077380A2 divulgue une poudre de granules de zircone et d'alumine destinés à la fabrication de pièces frittées céramiques, ladite poudre comprenant un stabilisant de la zircone.

Il existe donc un besoin pour un produit céramique fritté présentant un meilleur compromis dureté, ténacité et module à la rupture.

Un but de l'invention est de répondre, au moins partiellement, à ce besoin.

### Résumé de l'invention

L'invention propose un produit fritté présentant une analyse chimique telle que, en pourcentages en masse sur la base des oxydes,
- ZrO₂ partiellement stabilisée avec CeO₂ et Y₂O₃ : complément à 100%,
- Al₂O₃: > 10% et < 19%,
- additif choisi parmi CaO, les oxydes de manganèse, ZnO, SrO, les oxydes de cuivre, BaO, les oxydes de fer, et leurs mélanges : 0,2 - 6%,
- impuretés : < 2%, CeO₂ et Y₂O₃ étant présents en des quantités telles que, en pourcentage molaire sur la base de la somme de ZrO₂, CeO₂ et Y₂O₃,
- CeO₂ : ≥ 2,5 mol% et < 5,5 mol% et
- Y₂O₃ : 0,5 - 2 mol%.

De préférence, la distribution granulométrique du produit fritté est telle que :
- la taille moyenne des grains présentant un facteur de forme inférieur à 2,5, ou "grains ramassés", est inférieure à 2 µm,
- la longueur moyenne des nodules allongés alumineux est inférieure à 20 µm, un nodule allongé alumineux étant une structure présentant un facteur de forme supérieur ou égal à 2,5 et constituée d'un grain alumineux ou de plusieurs grains alumineux adjacents, un grain alumineux étant un grain constitué, pour plus de 40% de sa masse, d'Al₂O₃ et dudit additif.

Les inventeurs ont en particulier découvert qu'un excellent compromis entre la ténacité, la dureté et la contrainte à la rupture était possible en associant de faibles teneurs en oxyde de cérium et en alumine.

Un produit fritté selon l'invention peut être en particulier fabriqué suivant un procédé selon l'invention décrit ci-après.

Un produit fritté selon l'invention peut encore présenter une ou plusieurs des caractéristiques optionnelles suivantes :
- la teneur molaire en Y₂O₃ est de préférence inférieure à 1,9%, de préférence inférieure à 1,7%, de préférence inférieure à 1,5%, et/ou de préférence supérieure à 0,6%, de préférence supérieure à 0,8%, de préférence supérieure à 1%, en pourcentage molaire sur la base de la somme de ZrO₂, CeO₂ et Y₂O₃ ;
- la teneur en alumine Al₂O₃ est de préférence inférieure à 16%, et/ou de préférence supérieure à 11%, de préférence supérieure à 12%, de préférence supérieure à 13%, en pourcentage massique sur la base des oxydes ;
- la teneur molaire en CeO₂ est de préférence inférieure à 5%, de préférence inférieure à 4,5%, de préférence inférieure à 4,2%, de préférence inférieure à 4%, de préférence inférieure à 3,8%, et/ou supérieure à 3%, en pourcentage molaire sur la base de la somme de ZrO₂, CeO₂ et Y₂O₃ ;
- l'additif est de préférence choisi parmi CaO, les oxydes de manganèse, SrO, BaO et leurs mélanges, de préférence parmi CaO, les oxydes de manganèse, et leurs mélanges ;
- de préférence, l'additif est un mélange de CaO d'une part et d'un un plusieurs oxyde(s) de manganèse d'autre part ;
- la teneur en additif est de préférence supérieure à 0,3%, de préférence supérieure à 0,4%, de préférence supérieure à 0,5%, et/ou de préférence inférieure à 5%, de préférence inférieure à 4%, en pourcentage en masse sur la base des oxydes ;
- dans un mode de réalisation, l'additif comporte CaO, la teneur en CaO étant inférieure à 4%, de préférence inférieure à 3%, de préférence inférieure à 2%, de préférence inférieure à 1%, voire inférieure à 0,8%, voire inférieure à 0,6%, en pourcentage en masse sur la base des oxydes ;
- dans un mode de réalisation préféré, l'additif est un mélange d'un ou plusieurs oxyde(s) de manganèse et de CaO, la teneur en oxyde(s) de manganèse exprimée sous la forme MnO étant supérieure à 0,2%, de préférence supérieure à 0,3%, et/ou de préférence inférieure à 4%, de préférence inférieure à 3%, de préférence inférieure à 2%, de préférence inférieure à 1%, de préférence inférieure à 0,8%, et la teneur en CaO étant de préférence supérieure à 0,2%, et/ou inférieure à 4%, de préférence inférieure à 3%, de préférence inférieure à 2%, de préférence inférieure à 1%, de préférence inférieure à 0,8%, de préférence inférieure à 0,5%, en pourcentage en masse sur la base des oxydes ;
- la teneur en impuretés est de préférence inférieure à 1,0%, de préférence inférieure à 0,8%, de préférence inférieure à 0,5%, voire inférieure à 0,3%, en pourcentage massique sur la base des oxydes ; Dans un mode de réalisation, les impuretés sont constituées d'oxydes ;
- dans un mode de réalisation,
   - la teneur en alumine est supérieure à 10%, de préférence supérieure à 11%, de préférence supérieure à 12%, de préférence supérieure à 13%, et inférieure à 19%, de préférence inférieure à 16%, et
   - la teneur en ZrO₂ partiellement stabilisée avec CeO₂ et Y₂O₃ représente le complément à 100%,
      CeO₂ et Y₂O₃ étant présents en des quantités telles que, en pourcentage molaire sur la base de la somme de ZrO₂, CeO₂ et Y₂O₃,
      - la teneur molaire en Y₂O₃ est inférieure à 2%, de préférence inférieure à 1,9%, de préférence inférieure à 1,7%, de préférence inférieure à 1,5% et supérieure à 0,6%, de préférence supérieure à 0,8%, de préférence supérieure à 1%, et
      - la teneur molaire en CeO₂ est inférieure à 5,5%, de préférence inférieure à 5%, de préférence inférieure à 4,5%, de préférence inférieure à 4,2%, de préférence inférieure à 4%, de préférence inférieure à 3,8% et supérieure à 3%, et
   - la teneur en additif est supérieure à 0,2%, de préférence supérieure à 0,3%, de préférence supérieure à 0,4%, voire supérieure à 0,5% ou supérieure à 0,6%, et inférieure à 5%, de préférence inférieure à 4%, de préférence inférieure à 3%, de préférence inférieure à 2,5%, de préférence inférieure à 2%, voire inférieure à 1,5%, voire inférieure à 1%, en pourcentage en masse sur la base des oxydes, et
   - la teneur en impuretés est inférieure à 1,0%, de préférence inférieure à 0,8%, de préférence inférieure à 0,5%, voire inférieure à 0,3%, en pourcentage massique sur la base des oxydes ;
- Dans un mode de réalisation préféré,
   - la teneur en alumine est supérieure à 10%, de préférence supérieure à 11%, de préférence supérieure à 12%, de préférence supérieure à 13%, et inférieure à 19%, de préférence inférieure à 16%, et
   - la teneur en ZrO₂ partiellement stabilisée avec CeO₂ et Y₂O₃ représente le complément à 100%,
      CeO₂ et Y₂O₃ étant présents en des quantités telles que, en pourcentage molaire sur la base de la somme de ZrO₂, CeO₂ et Y₂O₃,
   - la teneur molaire en Y₂O₃ est inférieure à 2%, de préférence inférieure à 1,9%, de préférence inférieure à 1,7%, de préférence inférieure à 1,5%, et supérieure à 0,6%, de préférence supérieure à 0,8%, de préférence supérieure à 1%, et
   - la teneur molaire en CeO₂ est inférieure à 5,5%, de préférence inférieure à 5%, de préférence inférieure à 4,5%, de préférence inférieure à 4,2%, de préférence inférieure à 4%, de préférence inférieure à 3,8%, et supérieure à 3%, et
   - l'additif est un mélange d'un oxyde de manganèse et de CaO, la teneur en oxyde de manganèse exprimée sous la forme MnO étant supérieure à 0,2%, de préférence supérieure à 0,3% et inférieure à 4%, de préférence inférieure à 3%, de préférence inférieure à 2%, de préférence inférieure à 1%, de préférence inférieure à 0,8%, et la teneur en CaO étant supérieure à 0,2% et inférieure à 4%, de préférence inférieure à 3%, de préférence inférieure à 2%, de préférence inférieure à 1%, de préférence inférieure à 0,8%, de préférence inférieure à 0,5%, en pourcentage en masse sur la base des oxydes, et
   - la teneur en impuretés est inférieure à 1,0%, de préférence inférieure à 0,8%, de préférence inférieure à 0,5%, voire inférieure à 0,3%, en pourcentage massique sur la base des oxydes ;
- la taille moyenne des grains ramassés est inférieure à 1,5 µm, de préférence inférieure à 1 µm, de préférence inférieure à 0,5 µm et/ou de préférence supérieure à 0,1 µm, de préférence supérieure à 0,2 µm ;
- dans un mode de réalisation préféré, plus de 95%, de préférence plus de 97%, de préférence plus de 99% en nombre des grains ramassés sont des grains de zircone partiellement stabilisée et/ou des grains constitués pour plus de 40% de leur masse d'alumine ;
- la longueur moyenne des nodules allongés alumineux est inférieure à 18 µm, de préférence inférieure à 15 µm, voire inférieure à 10 µm et/ou supérieure à 1 µm, de préférence supérieure à 2 µm, de préférence supérieure à 5 µm ;
- plus de 50%, de préférence plus de 60%, de préférence plus de 70%, de préférence plus de 80% en nombre des nodules allongés alumineux présentent un facteur de forme supérieur ou égal à 3, voire supérieur ou égal à 4 ;
- le produit fritté présente un rapport H, égal au rapport de la surface couverte par les nodules allongés alumineux sur la surface couverte par lesdits nodules allongés alumineux et les grains ramassés comportant plus de 40% en masse d'alumine, exprimé en pourcentages, supérieur à 5%, de préférence supérieur à 10%, de préférence supérieur à 20%, et/ou de préférence inférieur à 95%, de préférence inférieur à 90%, de préférence inférieur à 80% ;
- plus de 30%, plus de 40%, plus de 60%, plus de 80%, plus de 90% en nombre des nodules allongés alumineux présentent une forme générale rectiligne ;
- lesdits nodules allongés alumineux comportent l'élément Al et les cations métalliques des oxydes ajoutés à titre d'additif (Ca et/ou Mn et/ou Zn et/ou Sr et/ou Cu et/ou Ba et/ou Fe).
- la masse volumique apparente du produit fritté est de préférence supérieure à 5,4 g/cm³, voire supérieure à 5,5 g/cm³, voire supérieure à 5,6 g/cm³ et/ou de préférence inférieure à 6,2 g/cm³, voire inférieure à 6,1 g/cm³, voire inférieure à 6 g/cm³, voire inférieure à 5,8, g/cm³ ;
- la masse volumique relative du produit fritté est de préférence supérieure à 95%, de préférence supérieure à 97%, de préférence supérieure à 98%, de préférence supérieure à 99%.

Il est aussi décrit un procédé de fabrication d'un produit fritté selon l'invention, comportant les étapes suivantes :
a) préparation d'une charge de départ comportant un mélange particulaire présentant une taille médiane inférieure à 1,0 µm, et dont la composition est adaptée de manière à obtenir, à l'issue de l'étape c), un produit fritté selon l'invention,
b) mise en forme de la charge de départ de manière à obtenir une préforme,
c) frittage de la préforme à une température de frittage supérieure à 1300°C de manière à obtenir un produit fritté selon l'invention.

Le procédé peut encore comporter une ou plusieurs des caractéristiques optionnelles suivantes :
- à l'étape a), on effectue une étape de broyage, de préférence par cobroyage, de manière à obtenir une taille médiane de préférence inférieure à 0,8 µm, de préférence inférieure à 0,6 µm, de préférence inférieure à 0,5 µm, voire inférieure à 0,3 µm, voire inférieure à 0,2 µm ;
- le procédé comporte de préférence, à l'étape b) une mise en forme par coulage en bande ou par pressage, de préférence par pressage uni axial, par pressage à chaud ou par pressage isostatique ;
- à l'étape c), la température de frittage est de préférence inférieure à 1600°C, de préférence inférieure à 1550°C, de préférence inférieure à 1500°C et/ou supérieure à 1350°C, de préférence supérieure à 1400°C.

L'invention concerne également un mélange particulaire comportant des particules de ZrO₂, des particules d'Al₂O₃, des particules de CeO₂, des particules d'Y₂O₃, et des particules de CaO et/ou des particules d'un ou plusieurs oxyde(s) de manganèse et/ou des particules de ZnO et/ou des particules de SrO et/ou des particules d'un ou plusieurs oxyde(s) de cuivre et/ou des particules de BaO et/ou des particules d'un ou plusieurs oxyde(s) de fer et/ou des particules de précurseurs de ces oxydes et/ou des particules de plusieurs de ces oxydes et/ou précurseurs de ces oxydes, le mélange particulaire étant tel que défini selon la revendication 12.

Avantageusement, un tel mélange particulaire est prêt à l'emploi.

Un mélange particulaire selon l'invention peut en particulier être conditionné dans des sacs.

De préférence, l'oxyde de manganèse est choisi parmi MnO, MnO₂, Mn₂O₃, Mn₃O₄ et leurs mélanges. De préférence, l'oxyde de manganèse est choisi parmi MnO, Mn₃O₄ et leurs mélanges.

De préférence, l'oxyde de cuivre est CuO.

De préférence, l'oxyde de fer est choisi parmi FeO, Fe₂O₃ et leurs mélanges.

De préférence, ledit mélange particulaire comporte des particules de ZrO₂, d'Al₂O₃, de CeO₂, d'Y₂O₃, des particules de CaO et des particules d'un oxyde de manganèse, de préférence de MnO et/ou de Mn₃O₄, et/ou des particules de précurseurs de ces oxydes, et/ou des particules de plusieurs de ces oxydes et/ou précurseurs de ces oxydes.

De préférence, la taille médiane dudit mélange particulaire est inférieure à 1 µm, de préférence inférieure à 0,8 µm, de préférence inférieure à 0,6 µm, de préférence inférieure à 0,5 µm, voire inférieure à 0,3 µm, voire inférieure à 0,2 µm.

De préférence, la surface spécifique dudit mélange particulaire est inférieure à 20 m²/g, de préférence inférieure à 15 m²/g, et/ou de préférence supérieure à 5 m²/g.

L'invention concerne enfin un dispositif choisi parmi :
- une pièce mécanique d'usure, de préférence choisie dans le groupe formé par un obturateur et un siège d'obturateur d'une vanne, un rotor de pompe, un joint de pompe et un corps de pompe,
- un article dentaire, en particulier une prothèse de dent ou une pièce d'un appareil orthodontique,
- un connecteur de fibres optiques, en particulier un embout (ou « ferrule » en anglais) ou un manchon (ou « sleeve » en anglais),
- un article décoratif choisi dans le groupe formé par un bijou, une montre, un bracelet, un collier, une bague, une broche, une épingle de cravate, un sac à main, un téléphone, un meuble, un ustensile ménager, une poignée, un bouton, un placage, une partie visible d'un équipement de bien de consommation, une partie de monture de lunettes, un article de vaisselle et un cadre,
   ledit dispositif comportant un produit fritté selon l'invention ou fabriqué à partir d'un mélange particulaire selon l'invention.

### Définitions

- Par « particule », on entend un produit solide individualisé dans une poudre.
- On appelle « frittage » la consolidation par traitement thermique à plus de 1100°C d'un agglomérat granulaire, avec éventuellement une fusion, partiellement ou totale, de certains de ses constituants (mais pas de tous ses constituants).
- On appelle « taille médiane » d'une poudre, généralement notée D₅₀, la taille divisant les particules de cette poudre en première et deuxième populations égales en masse, ces première et deuxième populations ne comportant que des particules présentant une taille supérieure ou égale, ou inférieure respectivement, à la taille médiane. La taille médiane peut par exemple être mesurée à l'aide d'un granulomètre laser.
- On appelle « taille moyenne » des grains d'un produit fritté, la dimension mesurée selon une méthode de « Mean Linear Intercept ». Une méthode de mesure de ce type est décrite dans la norme ASTM E1382.
- Les oxydes de manganèse comprennent notamment MnO, Mn₂O₃, MnO₂ et Mn₃O₄.
- Les oxydes de fer comprennent notamment FeO, Fe₂O₃, Fe₃O₄.
- Les oxydes de cuivre comprennent notamment CuO et Cu₂O.
- Par « impuretés », on entend les constituants inévitables, introduits nécessairement avec les matières premières. En particulier les composés faisant partie du groupe des oxydes, nitrures, oxynitrures, carbures, oxycarbures, carbonitrures et espèces métalliques de sodium et autres alcalins, vanadium et chrome sont des impuretés. A titre d'exemples, on peut citer Na₂O ou MgO. En revanche, l'oxyde d'hafnium n'est pas considéré comme une impureté.
- HfO₂ n'est pas chimiquement dissociable de ZrO₂. Dans la composition chimique d'un produit comportant de la zircone, ZrO₂ désigne donc la teneur totale de ces deux oxydes. Cependant, selon la présente invention, HfO₂ n'est pas ajouté volontairement dans la charge de départ. HfO₂ ne désigne donc que les traces d'oxyde d'hafnium, cet oxyde étant toujours naturellement présent dans les sources de zircone à des teneurs généralement inférieures à 2%. Par souci de clarté, on peut donc désigner indifféremment la teneur en zircone et en traces d'oxyde d'hafnium par ZrO₂+HfO₂ ou par ZrO₂, on encore par « teneur en zircone ».
- Par « précurseur » d'un oxyde, on entend un constituant apte à fournir ledit oxyde lors de la fabrication d'un produit fritté selon l'invention. Par exemple, le carbonate de baryum BaCO₃ est un précurseur possible de BaO.
- On appelle « facteur de forme d'un grain ou d'un nodule », le rapport entre la plus grande dimension du grain ou du nodule, ou "longueur" et la plus grande dimension mesurée perpendiculairement à la direction de ladite plus grande dimension, ou "largeur". Ces dimensions sont mesurées dans un plan d'observation d'une coupe polie du produit fritté, classiquement sur des clichés de microscopie électronique de cette coupe.
- On appelle « nodule allongé », un nodule présentant un facteur de forme F supérieur ou égal à 2,5.
- Par « masse volumique absolue » d'un produit fritté selon l'invention, on entend la masse volumique absolue classiquement calculée à l'aide d'une loi des mélanges, à partir d'une analyse chimique dudit produit fritté selon l'invention, en considérant que la totalité des oxydes d'yttrium et de cérium stabilise la zircone, et sans prendre en compte les additifs et les impuretés. La masse volumique absolue de la zircone partiellement stabilisée à Y₂O₃ et CeO₂ est calculée selon l'enseignement du document « Phase transformation and lattice constants of zirconia solid solutions in the system Y2O3-CeO2-ZrO2 », Urabe et Al., Materials Science Forum Vols. 34-36 (1988) pp 147-152.
- Par « masse volumique relative » d'un produit, on entend le rapport égal à la masse volumique apparente divisée par la masse volumique absolue, exprimé en pourcentage.

Sauf mention contraire, tous les pourcentages relatifs à la composition d'un produit ou relatifs à une charge de départ sont des pourcentages massiques sur la base des oxydes et tous les pourcentages de CeO₂ et Y₂O₃ sont des pourcentages molaires sur la base de la somme de ZrO₂, CeO₂ et Y₂O₃.

Sauf mention contraire, toutes les moyennes sont des moyennes arithmétiques.

Le rapport de la surface moyenne des nodules allongés alumineux sur la surface moyenne des grains ramassés, et le rapport du nombre de grains ramassés sur le nombre de nodules allongés alumineux sont mesurés dans un plan d'observation d'une coupe polie du produit fritté, classiquement sur des clichés de microscopie électronique de cette coupe.

### Brève description des figures

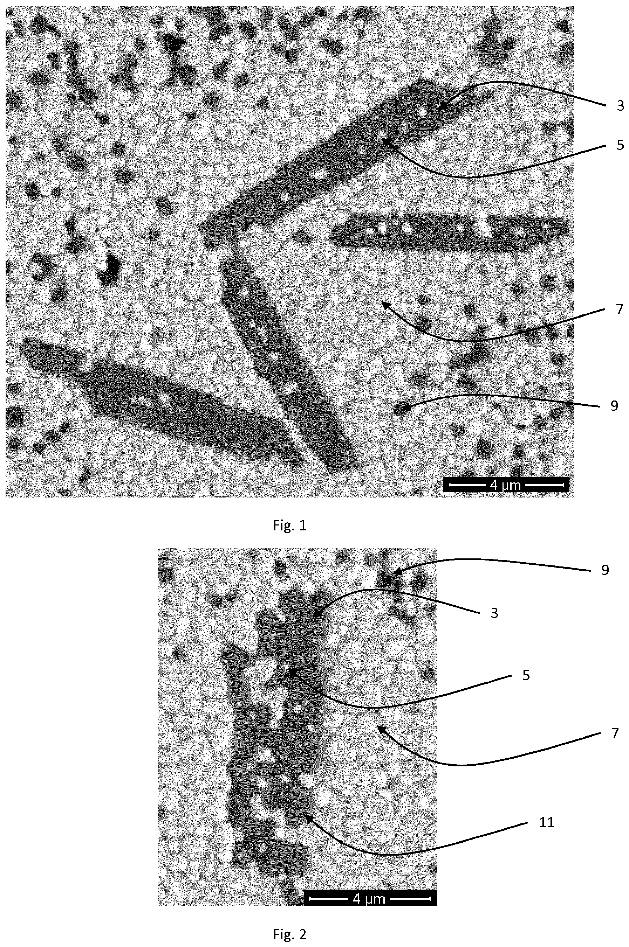

D'autres caractéristiques et avantages de l'invention apparaîtront encore à la lecture de la description détaillée qui va suivre et à l'examen du dessin annexé dans lequel les figures 1 et 2 représentent des photographies d'une coupe polie du produit fritté de l'exemple 12, selon l'invention, obtenu après frittage à une température de 1450°C, le produit fritté ayant subi, après polissage, une attaque thermique à 1400°C pendant 30 minutes pour révéler les joints de grains.

### Description détaillée

Pour fabriquer un produit fritté selon l'invention, on peut procéder suivant les étapes a) à c) décrites ci-dessus et détaillées ci-dessous.

**A l'étape a),** un broyage des matières premières peut être nécessaire pour obtenir une taille médiane, après mélange, inférieure à 1,0 µm.

En particulier, les poudres de matières premières apportant les oxydes peuvent être broyées individuellement ou, de préférence, cobroyées, si elles ne respectent pas la distribution granulométrique souhaitée, et en particulier si elles présentent une taille médiane supérieure à 1 µm, supérieure à 0,6 µm, supérieure à 0,5 µm, supérieure à 0,3 µm ou supérieure à 0,2 µm. Le broyage peut être réalisé en milieu humide, par exemple dans un broyeur à attrition. Après broyage en milieu humide, le mélange particulaire broyé est de préférence séché.

De préférence, à l'étape a), les poudres utilisées, notamment les poudres de ZrO₂, d'alumine Al₂O₃, de Y₂O₃, de CeO₂, et d'additif présentent chacune une taille médiane inférieure à 5 µm, inférieure à 3 µm, inférieure à 1 µm, inférieure à 0,7 µm, de préférence inférieure à 0,6 µm, de préférence inférieure à 0,5 µm. Avantageusement, lorsque que chacune de ces poudres présente une taille médiane inférieure à 1 µm, de préférence inférieure à 0,8 µm, de préférence inférieure à 0,6 µm, de préférence inférieure à 0,5 µm, voire inférieure à 0,3 µm, voire inférieure à 0,2 µm, le broyage est optionnel.

La mise en oeuvre de poudres présentant une faible taille médiane permet également, avantageusement, de réduire la température de frittage.

Ces poudres peuvent également être remplacées, au moins partiellement, par des poudres de précurseurs de ces oxydes, introduits dans des quantités équivalentes. De préférence, la poudre de zircone utilisée présente une aire spécifique, calculée par la méthode BET, supérieure à 5 m²/g, de préférence supérieure à 6 m²/g, de préférence supérieure à 7 m²/g, et inférieure à 20 m²/g, de préférence inférieure à 15 m²/g. Avantageusement, la température de frittage à l'étape d) est réduite, et le broyage, généralement en suspension, et la mise en suspension en sont facilitées.

L'ajout de CaO, et/ou d'un oxyde de manganèse, et/ou de ZnO, et/ou de SrO, et/ou d'un oxyde de cuivre, et/ou de BaO, et/ou d'un oxyde de fer et/ou de précurseurs de ces oxydes permet avantageusement d'augmenter la quantité de nodules allongés alumineux contenus dans le produit fritté et d'améliorer les performances mécaniques.

Les poudres apportant les oxydes ou les précurseurs sont de préférence choisies de manière que la teneur totale en impuretés soit inférieure à 2%, en pourcentage massique sur la base des oxydes.

Dans un mode de réalisation, Y₂O₃ est introduit au moins en partie sous la forme d'une zircone partiellement stabilisée à l'oxyde d'yttrium.

Dans un mode de réalisation, CeO₂ est introduit au moins en partie sous la forme d'une zircone partiellement stabilisée à l'oxyde de cérium, voire stabilisée à l'oxyde de cérium.

Comme cela est bien connu de l'homme du métier, la charge de départ peut comporter, en plus du mélange particulaire, un solvant et/ou un additif organique de mise en forme et/ou un dispersant, dont les natures et les quantités sont adaptées à la méthode de mise en forme de l'étape b).

De préférence le solvant est l'eau.

L'additif organique de mise en forme peut être choisi parmi les polyéthylènes glycol (ou PEG), les alcools polyvinyliques (ou APV), les latex, les dérivés de la cellulose et leurs mélanges.

Le dispersant peut être par exemple un polyacrylate.

Tous ces éléments disparaissent pendant les étapes de fabrication ultérieures, en pouvant laisser toutefois subsister quelques traces.

**A l'étape b),** la charge de départ est mise en forme par toute technique connue de l'homme du métier, de préférence par coulage en bande ou par pressage, de préférence par pressage uni axial, pressage à chaud ou par pressage isostatique. Dans le cas où la charge de départ est mise en forme par pressage une étape préalable de séchage, par exemple par atomisation, peut être réalisée. La taille des atomisats peut par exemple être comprise entre 20 µm et 250 µm.

Optionnellement, la mise en forme comporte un séchage de la préforme.

**A l'étape c),** la préforme est frittée à une température supérieure à 1300°C, de préférence supérieure à 1350°C, de préférence supérieure à 1400°C, de manière à obtenir un produit fritté selon l'invention. De préférence, la température de frittage est inférieure à 1600°C, de préférence inférieure à 1550°C, de préférence inférieure à 1500°C. Le frittage est effectué de préférence sous air à pression atmosphérique.

De préférence, la durée de frittage est supérieure à 1 heure, supérieure à 2 heures, et/ou inférieure à 10 heures, inférieure à 7 heures, ou inférieure à 5 heures. De préférence, la durée de frittage est comprise entre 2 et 5 heures.

La température de frittage est de préférence d'autant plus élevée que la quantité d'alumine est importante.

Les inventeurs ont relevé la présence d'une microstructure particulière dans les produits frittés selon l'invention.

Comme représentée sur la figure 1, la microstructure se caractérise par la présence de nodules allongés alumineux 3, qui peuvent se présenter sous la forme de baguettes sensiblement rectilignes. La figure 1 montre également des grains en inclusion 5, en particulier des grains de zircone, au sein des nodules allongés alumineux. La longueur moyenne des nodules allongés alumineux est typiquement supérieure 1 µm à et/ou inférieure à 20 µm. La microstructure spécifique aux produits selon l'invention comporte également des grains ramassés. Les grains ramassés présentent typiquement une taille moyenne inférieure à 2 µm et/ou supérieure à 0,1µm.

Un nodule allongé alumineux 3 peut être constitué par un grain, comme sur la figure 1 ou par un amas de grains alumineux 11 adjacents, comme sur la figure 2. Les grains alumineux 11 ont « coalescés » lors du frittage. Un grain alumineux est de préférence constitué, pour plus de 50%, plus de 60%, voire plus de 70% de sa masse, de Al₂O₃ et dudit additif.

Typiquement, plus de 90%, plus de 95%, voire plus de 98% ou 100% de la masse de la zircone se présente sous la forme de grains ramassés de zircone 7. Les inventeurs ont relevé que plus de 60%, de préférence plus de 80%, de préférence encore plus de 90% du volume de la zircone se présente sous la phase tétragonale.

CeO₂ et Y₂O₃ servent à stabiliser la zircone mais peuvent aussi être présents en dehors de celle-ci.

De préférence, plus de 90%, plus de 95%, plus de 98%, voire sensiblement 100% des autres grains ramassés sont des grains constitués pour plus de 40% de leur masse d'alumine 9.

Une analyse a montré que les nodules allongés alumineux 3 comportent de l'aluminium et les cations métalliques des oxydes ajoutés à titre d'additif (Ca et/ou Mn et/ou Zn et/ou Sr et/ou Cu et/ou Ba et/ou Fe). Lesdits nodules allongés alumineux peuvent également comporter l'élément Cérium (Ce). Ainsi, si l'additif comporte CaO et un oxyde de manganèse, lesdits nodules allongés alumineux comportent les éléments Al, Ca, Mn et Ce.

Les inventeurs ont constaté que les nodules allongés alumineux sont sensiblement constitués, en fonction de l'additif, d'une phase de type hibonite et/ou d'une phase de type magnéto-plombite.

Le rapport de la surface moyenne des nodules allongés alumineux sur la surface moyenne des grains ramassés est de préférence supérieur à 5, supérieur à 10, supérieur à 20, supérieur à 30, et/ou inférieur à 200, inférieur à 150, inférieur à 100.

Le rapport du nombre de grains ramassés sur le nombre de nodules allongés alumineux est de préférence supérieur à 10, supérieur à 20, supérieur à 30, supérieur à 40, supérieur à 50, et/ou inférieur à 2000, inférieur à 1500, inférieur à 1000, inférieur à 500.

### Exemples

Les exemples non limitatifs suivants sont donnés dans le but d'illustrer l'invention.

Des produits frittés ont été préparés à partir :
- d'une poudre de zircone yttriée contenant une teneur molaire en Y₂O₃ égale à 3%, présentant une aire spécifique de l'ordre de 10 m²/g et une taille médiane inférieure à 0,3 µm pour l'exemple 1,
- d'une poudre de zircone de pureté supérieure à 99%, présentant une aire spécifique de l'ordre de 10 m²/g et une taille médiane inférieure à 0,3 µm pour l'exemple 2,
- d'une poudre de zircone yttriée contenant une teneur molaire en Y₂O₃ égale à 1,2%, présentant une aire spécifique de l'ordre de 8 m²/g et une taille médiane inférieure à 5 µm pour les exemples 3 à 16,
- d'une poudre de CeO₂ de pureté supérieure à 99% et présentant une taille médiane inférieure à 10 µm pour les exemples 2 à 16,
- d'une poudre d'alumine de pureté supérieure à 99% et présentant une taille médiane inférieure à 0,5 µm pour les exemples 1 à 16,
- d'une poudre d'oxydes de manganèse, principalement sous la forme Mn₃O₄ et contenant également MnO, de pureté, exprimée sous la forme MnO, supérieure à 88%, et dont plus de 90% en masse des particules présentant une taille inférieure à 44 µm, pour les exemples 3 à 16,
- d'une poudre de carbonate de calcium présentant une taille médiane égale à 5 µm pour les exemples 3 à 16.

Ces poudres ont été mélangées puis co-broyées en milieu humide jusqu'à obtention d'un mélange particulaire présentant une taille médiane de particules inférieure à 0,3 µm. De l'alcool polyvinylique a ensuite été ajouté en une quantité égale à 2% sur la base de la matière sèche du mélange particulaire. La charge de départ obtenue a ensuite été atomisée sous la forme d'une poudre d'atomisats présentant une taille médiane égale à 60 µm, une masse volumique relative comprise entre 30% et 60% et un indice de sphéricité supérieur à 0,85 dans un sécheur-atomisateur, la masse volumique relative d'une poudre d'atomisats étant le rapport égal à la masse volumique réelle divisée par la masse volumique absolue, exprimé en pourcentage ; la masse volumique absolue d'une poudre d'atomisats étant le rapport égal à la masse de matière sèche de ladite poudre après un broyage à une finesse telle qu'il ne demeure sensiblement aucun pore fermé, divisée par le volume de cette masse après broyage, mesurée par pycnométrie à hélium, et la masse volumique réelle d'une poudre d'atomisats étant la moyenne des masses volumiques apparentes de chaque atomisat de la poudre, la masse volumique apparente d'un atomisat étant le rapport égal à la masse dudit atomisat divisée par le volume qu'occupe ledit atomisat.

A l'étape b), chaque poudre d'atomisats a ensuite été pressée sur une presse uni axiale à une pression égale à 100 MPa.

A l'étape c), les préformes obtenues ont ensuite été transférées dans un four de frittage où elles ont été portées, à une vitesse de 100°C/h, jusqu'à 1450°C. La température de 1450°C a été maintenue pendant 2 heures. La descente en température a été effectuée par refroidissement naturel.

### Protocoles de mesure

La dureté des produits frittés est mesurée à partir d'indentations Vickers à 0,3 kg. Après mesure de la longueur des fissures radiales, la ténacité a été calculée en utilisant la formule universelle développée par Liang et al. (« Evaluation by indentation of fracture toughness of ceramic materials », 1990).

Le module de rupture en flexion 3 points est mesuré sur les produits frittés dans les conditions de la norme ISO 6872.

La masse volumique apparente des produits frittés est mesurée par pesée hydrostatique.

L'analyse chimique des produits frittés est mesurée par « *Inductively Coupled Plasma »* ou ICP pour les éléments dont la quantité ne dépasse pas 0,5%. Pour déterminer la teneur des autres éléments, une perle du produit à analyser est fabriquée en fondant le produit, puis l'analyse chimique est réalisée par fluorescence X.

Le facteur de forme des grains et des nodules des produits frittés, la longueur moyenne des nodules allongés alumineux et le rapport H égal au rapport de la surface couverte par les nodules allongés alumineux sur la surface couverte par lesdits nodules allongés alumineux et les grains comportant plus de 40% en masse d'alumine, sont mesurés sur des images obtenues par microscopie électronique à balayage en électrons rétrodiffusés, d'échantillons de produits frittés, lesdites sections ayant préalablement été polies jusqu'à obtention d'une qualité miroir puis attaquées thermiquement pour révéler les joints de grains, dans un cycle présentant une vitesse de montée en température égale à 100°C/h, à une température de palier inférieure de 50°C à la température de frittage, maintenue pendant 30 minutes, et une descente en température en refroidissement naturel. Le grossissement utilisé pour la prise des images est choisi de façon à visualiser entre 2 et 4 nodules allongés alumineux sur une image. 20 images par produit fritté ont été réalisées.

La taille moyenne des grains des produits frittés ramassés a été mesurée par la méthode de « Mean Linear Intercept ». Une méthode de ce type est décrite dans la norme ASTM E1382. Suivant cette norme, on trace des lignes d'analyse sur des images des produits frittés, puis, le long de chaque ligne d'analyse, on mesure les longueurs, dites « intercepts », entre deux joints de grains ramassés consécutifs coupant ladite ligne d'analyse. Les lignes d'analyse sont déterminées de manière à ne pas couper de nodule allongé alumineux.

On détermine ensuite la longueur moyenne « l' » des intercepts « I».

Pour les tests ci-dessous, les intercepts ont été mesurés sur des images, obtenues par microscopie électronique à balayage, d'échantillons de produits frittés, lesdites sections ayant préalablement été polies jusqu'à obtention d'une qualité miroir puis attaquées thermiquement, à une température inférieure de 50°C à la température de frittage, pour révéler les joints de grains. Le grossissement utilisé pour la prise des images est choisi de façon à visualiser environ 100 grains ramassés sur une image. 5 images par produit fritté ont été réalisées.

La taille moyenne « d » des grains d'un produit fritté est donnée par la relation : d =1,56.I'. Cette formule est issue de la formule (13) de « Average Grain Size in Polycrystalline Ceramics » M. I. Mendelson, J. Am. Cerm. Soc. Vol. 52, No.8, pp443-446.

L'aire spécifique est mesurée par la méthode BET (Brunauer Emmet Teller) telle que décrite dans Journal of American Chemical Society 60 (1938), pages 309 à 316.

Le tableau 1 suivant résume les résultats obtenus.

**Tableau 1**

| Ex | base somme ZrO₂ + CeO₂ + Y₂O₃ | | | analyse chimique (% massique) | | | | | taille moyenne des grains ramassés (µm) | longueur moyenne des nodules allongés alumineux (µm) | rapport H (%) | masse volumique apparente (g/cm³) | masse volumique relative (%) | Dureté Vickers | ténacité (MPa.m ^{1/2}) | module de rupture en flexion 3 points (MPa) |
|---|---|---|---|---|---|---|---|---|---|---|---|---|---|---|---|---|
| | (% molaire) | | | | | | | | | | | | | | | |
| | ZrO₂ | CeO₂ | Y₂O₃ | ZrO₂ en partie stabilisée | Al₂O₃ | Additifs | | Impuretés | | | | | | | | |
| | | | | | | Oxyde de manganèse exprimé sous la forme MnO | CaO | | | | | | | | | |
| 1(*) | 97 | 0 | 3 | 79,7 | 20,0 | 0 | 0 | 0,3 | < 1 | - | 0 | 5,39 | 98,1 | 1430 | 8,5 | 780 |
| 2 (*) | 88 | 12 | 0 | 97,6 | 2,0 | 0 | 0 | 0,4 | < 1 | - | 0 | 6,08 | 97,1 | 780 | 11 | 560 |
| 3 (*) | 94,7 | 4,1 | 1,2 | 73,9 | 24,9 | 0,4 | 0,3 | 0,5 | < 1 | 6,0 | 16 | 5,35 | 99,80 | 1390 | 8,7 | |
| 4 | 94,9 | 4 | 1,1 | 83,8 | 14,9 | 0,6 | 0,3 | 0,4 | 0,33 | 7,6 | 37 | 5,63 | 99,87 | 1290 | 13,9 | 860 |
| 5 (*) | 93,1 | 5,8 | 1,1 | 98,7 | 0,3 | 0,5 | 0,3 | 0,2 | < 1 | 7,7 | > 95 | 6,11 | 99,78 | 1180 | 13,9 | |
| 6 (*) | 93,8 | 5,1 | 1,1 | 95,9 | 3,0 | 0,4 | 0,3 | 0,4 | 0,40 | 9,1 | > 95 | 6,02 | 99,94 | 1180 | 14,4 | |
| 7 (*) | 97 | 1,8 | 1,2 | 88,8 | 10,1 | 0,5 | 0,2 | 0,4 | < 1 | 7,4 | 25 | 5,7 | 98,90 | 1170 | 15,1 | |
| 8 (*) | 94,9 | 4 | 1,1 | 94 | 5,0 | 0,5 | 0,3 | 0,2 | < 1 | 6,8 | 84 | 5,92 | 99,59 | 1160 | 16,2 | |
| 9 (*) | 96,8 | 2 | 1,2 | 83,9 | 14,9 | 0,5 | 0,3 | 0,4 | < 1 | 6,2 | 33 | 5,463 | 97,23 | 1130 | 8,2 | |
| 10 | 94 | 4,9 | 1,1 | 81,1 | 16,9 | 0,6 | 0,3 | 1,1 | 0,36 | 6,4 | 27 | 5,58 | 99,81 | 1340 | 12,8 | 740 |
| 11 | 94 | 4,9 | 1,1 | 88,4 | 10,1 | 0,5 | 0,3 | 0,7 | 0,35 | 6,1 | 58 | 5,78 | 99,72 | 1220 | 12,9 | 780 |
| 12 | 93 | 5,9 | 1,1 | 88,1 | 10,5 | 0,4 | 0,3 | 0,7 | 0,44 | 6,5 | 58 | 5,76 | 99,47 | 1260 | 12,7 | 720 |
| 13 (*) | 91,4 | 7,5 | 1,1 | 88,7 | 10,2 | 0,4 | 0,4 | 0,3 | < 1 | 5,2 | 59 | 5,75 | 98,83 | 1180 | 11,1 | |
| 14 (*) | 89,9 | 9 | 1,1 | 89,2 | 10,0 | 0,4 | 0,3 | 0,1 | < 1 | 6,5 | 62 | 5,79 | 99,16 | 1170 | 8,3 | |
| 15 | 95,5 | 3,4 | 1,1 | 84,7 | 14,1 | 0,5 | 0,3 | 0,4 | < 1 | 5,7 | 37 | 5,6 | 99,07 | 1280 | 14,1 | 860 |
| 16 (*) | 93,4 | 5,5 | 1,1 | 94 | 5,0 | 0,5 | 0,3 | 0,2 | < 1 | 6,8 | 92 | 5,94 | 99,64 | 1200 | 12,4 | 820 |

| | | | | | | | | | | | | | | | | |
|---|---|---|---|---|---|---|---|---|---|---|---|---|---|---|---|---|
| (*) : exemples hors invention | | | | | | | | | | | | | | | | |

Les inventeurs considèrent qu'il existe un bon compromis entre la dureté, la ténacité et le module de rupture en flexion 3 points lorsque :
- la dureté Vickers est supérieure ou égale à 1210, et
- la ténacité est supérieure ou égale à 10 MPa.m^{1/2}, et
- le module de rupture en flexion 3 points est supérieur ou égal à 700 MPa.

De préférence, la dureté est supérieure ou égale à 1250, et/ou la ténacité est supérieure ou égale à 11 MPa.m^{1/2}, de préférence supérieure ou égale à 12 MPa.m^{1/2}, de préférence supérieure ou égale à 13 MPa.m^{1/2}, de préférence supérieure ou égale à 14 MPa.m^{1/2}, et le module de rupture en flexion 3 points est supérieure ou égal à 750 MPa, de préférence supérieure à 800 MPa.

Les exemples 1 et 2, hors invention, montrent qu'un produit fritté comportant une zircone en partie stabilisée à 3% molaire d'Y₂O₃ et une teneur en alumine égale à 20%, et qu'un produit fritté comportant une zircone stabilisée à 12% molaire de CeO₂ et une teneur en alumine égale à 2%, respectivement, ne satisfont pas le compromis recherché.

Une comparaison de l'exemple 3, hors invention, et de l'exemple 4 montre la nécessité d'une teneur en alumine inférieure à 19%. Cette comparaison permet également de constater que pour des teneurs en oxyde de cérium faibles, l'augmentation de la quantité d'alumine au-delà de 19% conduit à une diminution brutale de la ténacité.

Les exemples 5 et 16 montrent cependant la nécessité d'une teneur minimale en alumine supérieure à 10%.

Les exemples 7 et 9, hors invention, montrent qu'une teneur molaire en CeO₂ égale à 1,8% et 2% respectivement, est trop faible et ne permet pas d'atteindre le compromis recherché.

Les exemples 13 et 14, hors invention, montrent qu'une teneur molaire en CeO₂ égale à 7,5% et 9% respectivement, est trop élevée et ne permet pas d'atteindre le compromis recherché. Les exemples 13 et 14 montrent également que, pour de faibles teneurs en alumine selon l'invention, la présence d'une quantité d'oxyde de cérium supérieure à 6,5 mol% conduit à une dureté insatisfaisante.

L'exemple 15 est l'exemple préféré d'entre tous. L'exemple 15 montre qu'il est particulièrement avantageux de limiter la teneur en oxyde de cérium à moins de 5%, à moins de 4%, et même à moins de 3,5%.

Comme cela apparaît clairement à présent, les inventeurs ont découvert que la présence simultanée d'une faible teneur en alumine et d'une faible teneur en oxyde de cérium permet avantageusement d'obtenir un produit fritté à base d'alumine et de zircone présentant un bon compromis entre dureté, ténacité et module de rupture.

Bien entendu, l'invention n'est pas limitée aux exemples et modes de réalisation décrits ci-dessus.

## Revendications

1. Produit fritté présentant une analyse chimique telle que, en pourcentages en masse sur la base des oxydes,
- ZrO₂ partiellement stabilisée avec CeO₂ et
Y₂O₃ : complément à 100%,
- Al₂O₃: > 10% et < 19%
- additif choisi parmi CaO, les oxydes de
manganèse, ZnO, SrO, les oxydes de
cuivre, BaO, les oxydes de fer, et leurs
mélanges : 0,2 - 6%,
- impuretés : < 2%,
CeO₂ et Y₂O₃ étant présents en des quantités telles que, en pourcentage molaire sur la base de la somme de ZrO₂, CeO₂ et Y₂O₃,
- CeO₂ : ≥ 2,5 mol% et < 5,5 mol%
- Y₂O₃ : 0,5 - 2 mol%.

2. Produit fritté selon la revendication précédente, dans lequel ladite teneur molaire en CeO₂ est inférieure 5% et supérieure à 3%.

3. Produit fritté selon la revendication immédiatement précédente, dans lequel ladite teneur molaire en CeO₂ est inférieure à 4%.

4. Produit fritté selon l'une quelconque des revendications précédentes, dans lequel ladite teneur molaire en Y₂O₃ est inférieure à 1,7% et supérieure à 1%.

5. Produit fritté selon l'une quelconque des revendications précédentes, dans lequel la teneur en alumine Al₂O₃ est supérieure à 11% et inférieure à 16%, en pourcentage massique sur la base des oxydes.

6. Produit fritté selon l'une quelconque des revendications précédentes, dans lequel la teneur en additif est supérieure à 0,5% et inférieure à 4%, en pourcentages en masse sur la base des oxydes.

7. Produit fritté selon l'une quelconque des revendications précédentes, dans lequel l'additif est un mélange de CaO d'une part et d'un ou plusieurs oxyde(s) de manganèse d'autre part.

8. Produit fritté selon la revendication immédiatement précédente, dans lequel la teneur en CaO est supérieure à 0,2% et inférieure à 1%, et dans lequel la teneur en oxyde(s) de manganèse exprimée sous la forme MnO est supérieure à 0,2% et inférieure 1%.

9. Produit fritté selon l'une quelconque des revendications précédentes, présentant une distribution granulométrique telle que la taille moyenne des grains présentant un facteur de forme inférieur à 2,5, ou "grains ramassés", est inférieure à 2 µm, et la longueur moyenne des nodules allongés alumineux est inférieure à 20 µm, un nodule allongé alumineux étant une structure présentant un facteur de forme supérieur ou égal à 2,5 et constituée d'un grain alumineux ou de plusieurs grains alumineux adjacents, un grain alumineux étant un grain constitué, pour plus de 40% de sa masse, d'Al₂O₃ et dudit additif.

10. Produit fritté selon la revendication précédente, dans lequel le rapport de la surface couverte par les nodules allongés alumineux sur la surface couverte par lesdits nodules allongés alumineux et par les grains ramassés comportant plus de 40% en masse d'alumine, exprimé en pourcentages, est supérieur à 5% et inférieur à 95%.

11. Produit fritté selon l'une quelconque des deux revendications immédiatement précédentes, dans lequel la longueur moyenne des nodules allongés alumineux est supérieure à 1 µm.

12. Mélange particulaire présentant une taille médiane inférieure à 1,0 µm, et comportant des particules de ZrO₂, des particules de Al₂O₃, des particules de CeO₂, des particules de Y₂O₃, et des particules d'un additif choisi parmi CaO, et/ou des particules d'un ou plusieurs oxyde(s) de manganèse et/ou des particules de SrO et/ou des particules de BaO, et/ou des particules de précurseurs de ces oxydes, et/ou des particules de plusieurs de ces oxydes et/ou précurseurs de ces oxydes, le mélange particulaire présentant une composition chimique telle qu'un procédé de fabrication comportant les étapes suivantes :
a) préparation d'une charge de départ comportant ledit mélange particulaire,
b) mise en forme de la charge de départ de manière à obtenir une préforme,
c) frittage de la préforme à une température de frittage supérieure à 1300°C,
conduit à un produit fritté selon l'une quelconque des revendications précédentes.

13. Mélange particulaire selon la revendication précédente, comportant des particules de ZrO₂, des particules d'Al₂O₃, des particules de CeO₂, des particules d'Y₂O₃, des particules de CaO et des particules d'un ou plusieurs oxyde(s) de manganèse, de préférence des particules de MnO et/ou des particules de de Mn₃O₄, et/ou des particules de précurseurs de ces oxydes, et/ou des particules de plusieurs de ces oxydes et/ou précurseurs de ces oxydes.

14. Dispositif choisi parmi :
- une pièce mécanique d'usure, de préférence choisie dans le groupe formé par un obturateur et un siège d'obturateur d'une vanne, un rotor de pompe, un joint de pompe et un corps de pompe,
- un article dentaire, en particulier une prothèse de dent ou une pièce d'un appareil orthodontique,
- un connecteur de fibres optiques, en particulier un embout ou un manchon,
- un article décoratif choisi dans le groupe formé par un bijou, une montre, un bracelet, un collier, une bague, une broche, une épingle de cravate, un sac à main, un téléphone, un meuble, un ustensile ménager, une poignée, un bouton, un placage, une partie visible d'un équipement de bien de consommation, une partie de monture de lunettes, un article de vaisselle et un cadre,
comportant un produit fritté selon l'une quelconque des revendications 1 à 11 ou fabriqué à partir d'un mélange particulaire selon l'une quelconque des deux revendications immédiatement précédentes.

## Patentansprüche

1. Sinterprodukt, das eine derartige chemische Analyse aufweist, dass, in Massenprozent auf der Basis der Oxide,
- ZrO₂, teilweise mit CeO₂ und Y₂O₃ stabilisiert: zu 100 % fehlender Anteil,
- Al₂O₃: > 10 % und < 19 %
- Zusatzstoff, gewählt unter CaO, den Manganoxiden, ZnO, SrO, den Kupferoxiden, BaO, den Eisenoxiden, und ihren Gemischen: 0,2 - 6 %,
- Verunreinigungen: < 2 %,
wobei CeO₂ und Y₂O₃ in derartigen Mengen vorkommen, dass, in Molprozent auf der Basis der Summe von ZrO₂, CeO₂ und Y₂O₃,
- CeO₂: ≥ 2,5 Mol-% und < 5,5 Mol-%
- Y₂O₃ : 0,5 - 2 Mol-%.

2. Sinterprodukt nach dem vorhergehenden Anspruch, bei dem der Molgehalt an CeO₂ kleiner als 5 % und größer als 3 % ist.

3. Sinterprodukt nach dem unmittelbar vorhergehenden Anspruch, bei dem der Molgehalt an CeO₂ kleiner als 4 % ist.

4. Sinterprodukt nach einem der vorhergehenden Ansprüche, bei dem der Molgehalt an Y₂O₃ kleiner als 1,7 % und größer als 1 % ist.

5. Sinterprodukt nach einem der vorhergehenden Ansprüche, bei dem der Gehalt an Aluminiumoxid Al₂O₃ größer als 11 % und kleiner als 16 % in Massenprozent auf der Basis der Oxide ist.

6. Sinterprodukt nach einem der vorhergehenden Ansprüche, bei dem der Zusatzstoffgehalt größer als 0,5 % und kleiner als 4 % in Massenprozent auf der Basis der Oxide ist.

7. Sinterprodukt nach einem der vorhergehenden Ansprüche, bei dem der Zusatzstoff ein Gemisch aus CaO einerseits und einem oder mehreren Manganoxid(en) andererseits ist.

8. Sinterprodukt nach dem unmittelbar vorhergehenden Anspruch, bei dem der Gehalt an CaO größer als 0,2 % und kleiner als 1 % ist und bei dem der Gehalt an Manganoxid(en), ausgedrückt in der Form MnO, größer als 0,2 % und kleiner als 1 % ist.

9. Sinterprodukt nach einem der vorhergehenden Ansprüche, das eine derartige Korngrößenverteilung aufweist, dass die mittlere Größe der Körner, die einen Formfaktor unter 2,5 aufweisen, oder der "gedrängten Körner" kleiner als 2 µm ist und die mittlere Länge der aluminiumhaltigen langgestreckten Knötchen kleiner als 20 µm ist, wobei ein aluminiumhaltiges langgestrecktes Knötchen eine Struktur ist, die einen Formfaktor größer oder gleich 2,5 aufweist und aus einem aluminiumhaltigen Korn oder aus mehreren angrenzenden aluminiumhaltigen Körnern besteht, wobei ein aluminiumhaltiges Korn ein Korn ist, das zu mehr als 40 % seiner Masse aus Al₂O₃ und dem Zusatzstoff besteht.

10. Sinterprodukt nach dem vorhergehenden Anspruch, bei dem das Verhältnis der durch die aluminiumhaltigen langgestreckten Knötchen bedeckten Fläche zu der Fläche, die durch die aluminiumhaltigen langgestreckten Knötchen und durch die gedrängten Körner, die mehr als 40 Massen-% Aluminiumoxid beinhalten, bedeckt wird, ausgedrückt in Prozent, größer als 5 % und kleiner als 95 % ist.

11. Sinterprodukt nach einem der beiden unmittelbar vorhergehenden Ansprüche, bei dem die mittlere Länge der aluminiumhaltigen langgestreckten Knötchen größer als 1 µm ist.

12. Partikelgemisch, aufweisend eine Mediangröße unter 1,0 µm und enthaltend Partikel von ZrO₂, Partikel von Al₂O₃, Partikel von CeO₂, Partikel von Y₂O₃ und Partikel eines Zusatzstoffs, der unter CaO und/oder Partikeln eines oder mehrerer Manganoxid(e) und/oder Partikeln von SrO und/oder Partikeln von BaO und/oder Partikeln von Vorläufern dieser Oxide und/oder Partikeln von mehreren dieser Oxide und/oder Vorläufern dieser Oxide gewählt ist, wobei das Gemisch eine derartige chemische Zusammensetzung aufweist, dass ein Herstellungsverfahren mit den folgenden Schritten:
a) Zubereiten eines Ansatzes, der das Partikelgemisch beinhaltet,
b) Informbringen des Ansatzes, so dass ein Vorformling erhalten wird,
c) Sintern des Vorformling bei einer Sintertemperatur über 1300 °C,
zu einem Sinterprodukt nach einem der vorhergehenden Ansprüche führt.

13. Partikelgemisch nach dem vorhergehenden Anspruch, beinhaltend Partikel von ZrO₂, Partikel von Al₂O₃, Partikel von CeO₂, Partikel von Y₂O₃, Partikel von CaO und Partikel von einem oder mehreren Manganoxid(en), bevorzugt Partikel von MnO und/oder Partikel von Mn₃O₄, und/oder Partikel von Vorläufern dieser Oxide und/oder Partikel von mehreren dieser Oxide und/oder Vorläufern dieser Oxide.

14. Vorrichtung, die gewählt ist unter:
- einem mechanischen Verschleißteil, das bevorzugt aus der Gruppe gewählt ist, die durch ein Verschlusselement und einen Verschlusselementsitz eines Ventils, einen Pumpenrotor, eine Pumpendichtung und ein Pumpengehäuse gebildet wird,
- einem Zahnartikel, insbesondere einer Zahnprothese oder einem Teil eines orthodontischen Geräts,
- einem Glasfaserverbinder, insbesondere einer Ferrule oder einer Hülse,
- einem dekorativen Artikel, der aus der Gruppe gewählt ist, die durch ein Schmuckstück, eine Uhr, ein Armband, eine Halskette, einen Ring, eine Brosche, eine Krawattennadel, eine Handtasche, ein Telefon, ein Möbelstück, ein Haushaltsgerät, einen Griff, einen Knopf, eine Plattierung, ein sichtbares Teil einer Vorrichtung eines Konsumguts, einem Brillenfassungsteil, einem Geschirrartikel und einem Rahmen gebildet wird,
die ein Sinterprodukt nach einem der Ansprüche 1 bis 11 beinhaltet oder aus einem Partikelgemisch nach einem der beiden unmittelbar vorhergehenden Ansprüche hergestellt ist.

## Claims

1. Sintered product having a chemical analysis such that, as percentages by weight on the basis of the oxides,
- ZrO₂ partially stabilized with CeO₂ and Y₂O₃ :
balance to 100%,
- Al₂O₃: > 10% and < 19%
- additive chosen from CaO, manganese oxides, ZnO, SrO, copper oxides, BaO, iron oxides, and mixtures thereof: 0.2 - 6%,
- impurities: < 2%,
CeO₂ and Y₂O₃ being present in amounts such that, as a molar percentage on the basis of the sum of ZrO₂, CeO₂ and Y₂O₃,
- CeO₂: ≥ 2.5 mol% and < 5.5 mol%
- Y₂O₃ : 0.5 - 2 mol%.

2. Sintered product according to the preceding claim, wherein said molar content of CeO₂ is less than 5% and greater than 3%.

3. Sintered product according to the immediately preceding claim, wherein said molar content of CeO₂ is less than 4%.

4. Sintered product according to any one of the preceding claims, wherein said molar content of Y₂O₃ is less than 1.7% and greater than 1%.

5. Sintered product according to any one of the preceding claims, wherein the content of alumina Al₂O₃ is greater than 11% and less than 16%, as a percentage by weight on the basis of the oxides.

6. Sintered product according to any one of the preceding claims, wherein the content of additive is greater than 0.5% and less than 4%, as a percentage by weight on the basis of the oxides.

7. Sintered product according to any one of the preceding claims, wherein the additive is a mixture of CaO on the one hand and of one or more manganese oxides on the other hand.

8. Sintered product according to the immediately preceding claim, wherein the content of CaO is greater than 0.2% and less than 1%, and wherein the content of manganese oxide(s) expressed in the form MnO is greater than 0.2% and less than 1%.

9. Sintered product according to any one of the preceding claims, having a particle size distribution such that the mean size of the grains having a shape factor of less than 2.5, or "compact grains", is less than 2 µm, and the mean length of the aluminous elongated nodules is less than 20 µm, an aluminous elongated nodule being a structure having a shape factor greater than or equal to 2.5 and formed of an aluminous grain or of several adjacent aluminous grains, an aluminous grain being a grain formed, for more than 40% of its weight, of Al₂O₃ and of said additive.

10. Sintered product according to the preceding claim, wherein the ratio of the surface covered by the aluminous elongated nodules to the surface covered by said aluminous elongated nodules and by the compact grains comprising more than 40% by weight of alumina, expressed as percentages, is greater than 5% and less than 95%.

11. Sintered product according to either one of the two immediately preceding claims, wherein the mean length of the aluminous elongated nodules is greater than 1 µm.

12. Particulate mixture having a median size less than 1.0 µm and comprising ZrO₂ particles, Al₂O₃ particles, CeO₂ particles, Y₂O₃ particles and particles of an additive chosen from CaO, and/or particles of one or more manganese oxides and/or SrO particles and/or BaO particles and/or particles of precursors of these oxides, and/or particles of several of these oxides and/or precursors of these oxides, the particulate mixture having a chemical composition such that a method of manufacture comprising the following steps of:
a) preparing a feedstock comprising said particulate mixture
b) shaping the feedstock so as to obtain a preform,
c) sintering the preform at a sintering temperature above 1300°C,
leads to a sintered product according to any one of the preceding claims.

13. Particulate mixture according to the preceding claim, comprising ZrO₂ particles, Al₂O₃ particles, CeO₂ particles, Y₂O₃ particles, CaO particles and particles of one or more manganese oxides, preferably MnO particles and/or Mn₃O₄ particles, and/or particles of precursors of these oxides, and/or particles of several of these oxides and/or precursors of these oxides.

14. Device chosen from:
- a mechanical wearing part, preferably chosen from the group formed by a closure member and a closure member seat of a valve, a pump rotor, a pump seal and a pump body,
- a dental article, in particular a tooth prosthesis or a part of an orthodontic appliance,
- an optical fiber connector, in particular a ferrule or a sleeve,
- a decorative article chosen from the group formed by a jewel, a watch, a bracelet, a necklace, a ring, a brooch, a tie pin, a handbag, a telephone, a piece of furniture, a household utensil, a handle, a button, a veneer, a visible part of a consumer goods item, a part of a spectacle frame, a piece of crockery and a frame,
comprising a sintered product according to any one of Claims 1 to 11 or manufactured from a particulate mixture according to either of the two immediately preceding claims.
